Europäisches Patentamt

European Patent Office

Office européen des brevets

(19)

(11) Veröffentlichungsnummer : **0 113 447**
**B1**

(12) **EUROPÄISCHE PATENTSCHRIFT**

(45) Veröffentlichungstag der Patentschrift :
10.02.88

(51) Int. Cl.⁴ : **B 01 D 13/00, C 07 K 3/26**

(21) Anmeldenummer : **83112350.0**

(22) Anmeldetag : **08.12.83**

(54) **Verfahren zur fraktionierten Auftrennung von Proteingemischen mittels Membranen.**

(30) Priorität : **09.12.82 DE 3245591**

(43) Veröffentlichungstag der Anmeldung : –
**18.07.84 Patentblatt 84/29**

(45) Bekanntmachung des Hinweises auf die Patenterteilung : **10.02.88 Patentblatt 88/06**

(84) Benannte Vertragsstaaten :
**AT BE CH DE FR GB IT LI LU NL SE**

(56) Entgegenhaltungen :
**DE-A- 2 248 804**
**DE-A- 2 412 118**
**DE-A- 2 510 309**
**DE-A- 2 628 063**
**US-A- 4 216 205**

(73) Patentinhaber : **Schott Glaswerke**
**Hattenbergstrasse 10**
**D-6500 Mainz (DE)**
**AT BE CH DE FR GB IT LI LU NL SE**
**Carl-Zeiss-Stiftung trading as SCHOTT GLASWERKE**
**Hattenbergstrasse 10**
**D-6500 Mainz 1 (DE)**
**GB**

(72) Erfinder : **Schnabel, Roland, Dr. Dipl.-Chem.**
**Breckenheimer Strasse 71**
**D-6238 Hofheim (DE)**
Erfinder : **von Baeyer, Hans, Dr.Med.**
**Spandauer Damm 130**
**D-1000 Berlin 19 (DE)**

(74) Vertreter : **Schmitz, Waldemar, Dipl.-Ing. et al**
**Büro Dr. J. Rasper Bierstadter Höhe 22**
**D-6200 Wiesbaden (DE)**

### Beschreibung

Verfahren zur Auftrennung von Stoffgemischen werden in der chemischen Verfahrenstechnik, der Biotechnologie und der Medizintechnik zur Reinigung, Konzentrierung und zur Gewinnung spezifischer Substanzen eingesetzt. Hierunter fallen die bekannten konventionellen Methoden wie Extraktion, Destillation, Gefriertrocknung, Filtration, Fällung und chromatographische Abtrennmethoden. In der Medizintechnik benutzt man zusätzlich in der Hämotologie das Zentrifugenverfahren zur Abtrennung fester Blutbestandteile vom Plasma.

Dieses letztgenannte Verfahren wird im allgemeinen als Plasmapherese bezeichnet und wurde 1959 entwickelt, Tullis, J. L. ; D. M. Surgenor ; R. J. Tinch ; M. D'Hont « New principle of closed system centrifugation », Science 24 (1956) 792. In den letzten 10 Jahren wurde dieses Verfahren sowohl in der Medizintechnik als auch in der Biotechnologie durch Membrantrennverfahren ergänzt. Dieses Verfahren erlaubt eine wesentlich schonendere Abtrennung der oft empfindlichen suspendierten Teilchen. Das Verfahren bezeichnet man als Membranplasmapherese in der Medizintechnik oder als Cross-Flow-Microfiltration in der technischen Chemie bzw. Biotechnologie, A. S. Michaels ; Desalination 35 (1980) 329.

Wenn auch dieses Verfahren einen wesentlichen Fortschritt in der schonenden Aufarbeitung von Suspensionen darstellt und die Konzentrierung bestimmter Substanzklassen aus Lösungen mit Hilfe von Membranen unter dem Begriff Ultrafiltration bzw. Hämofiltration ebenfalls bekannt ist, z. B. R. Schnabel ; Fette-Seifen-Anstrichmittel 81 Nr. 2 (1979) 83, so ist es bis heute schwierig, eine fraktionierte, selektive Auftrennung von Substanzgemischen zu erreichen. Der Frage der Selektivität kommt hierbei eine erhöhte Bedeutung zu, da durch den Einfluß von sogenannten Konzentrationspolarisationseffekten durch Sekundärmembranbildung und Wechselwirkung zwischen Membran und Spezies die auf Grund der Porengröße zu erwartende Trenngrenze verschoben wird, H. Chmiel ; Therapeutic Plasma Exchange ; Ed. H. J. Gurland, V. Heinze, H. A. Lee ; Springer (1981) 15.

In der zitierten Literaturstelle sind allein zur medizinischen Plasmapherese 560 Literaturstellen aufgelistet. In der Literatur wird auch zum Teil die Fraktionierung von Komponenten unterschiedlicher Molekulargewichts diskutiert, z. B. H. Strathmann ; Chemie Technik 11 Nr. 7 (1982) 813. Hierbei spricht man gleichgewichtig von Kaskaden-Filtration, fraktionierter Membranplasmapherese oder spezifischer Membranplasmapherese. Durch Hintereinanderschalten von Mikrofiltrationsmembran, Ultrafiltrationsmembran und Hyperfiltrationsmembran lassen sich makromolekulare Bestandteile von niedermolekularen Bestandteilen abtrennen.

Prinzipiell ist ein Membranmaterial, das sich für solche Prozesse eignet, in der DE-PS 2 454 111 beschrieben und die Verwendung dieses Materials zur Diafiltration von Blut in der DE-PS 2 757 673. Eine Kombination unterschiedlicher Membranen der beschriebenen Anordnung ist ebenfalls in der Literatur beschrieben, H. G. Sieberth ; in Plasma Exchange Symposiumband, Ed. H. G. Sieberth, Schattauer Verlag (1980) 29. Das Problem der dort beschriebenen Anordnung — die in der getesteten Form als nicht praktisch anwendbar beschrieben wird — ist in unterschiedlichen Punkten zu sehen :

Die Membranen weisen in vivo ein total unterschiedliches Verhalten gegenüber den in vitro gemessenen Werten auf. Die Albumin-Rückgewinnung war zu niedrig, so daß zusätzliche Substitution von Albumin durchgeführt wurde ;

Der Protein-Gehalt im Filtrat blieb nicht konstant. Um dies zu kompensieren, wurde dem Plasma Elektrolytlösung zur Aufrechterhaltung der Proteinkonzentration zugesetzt. Dieses stellt keine Verdünnung, sondern eine Substitution abfiltrierter Lösungsmittelmengen dar.

In den anderen Literaturstellen, z. B. J. Takeda ; Y. Ono ; K. Yagita ; Y. Suma ; K. Katoka ; Artificial Organs, Vol 5 (Suppl.) (1981) 168, wird diese Substitution zwar als Verdünnung beschriebenen, jedoch reicht der Verdünnungsfaktor nur bis zu einer höchstens dreifachen Verdünnung.

Die zusätzliche Substitution von Albumin, d. h. die zu geringe Rückgewinnungsrate niedermolekularer Substanzen, ist bei an sich technologisch optimierten Membranen darauf zurückzuführen, daß mit Filtrationsraten bis zu 40 % gearbeitet wird. Bei zusätzlicher Zurückhaltung dieser Substanzen durch die Membran oder auch durch die sich ausbildende Filterschicht können so, insgesamt gesehen, nur geringe Rückgewinnungsraten erzielt werden.

Aus der DE-A-2 412 118 ist ein Verfahren zur Isolierung einer Proteinfraktion aus einer wässrigen Lösung oder Suspension, welche diese Fraktion hochmolekularer Stoffe in Mischung mit anderen niedermolekularen Substanzen enthält bekannt. In dem vorgeschlagenen Verfahren wird ein Retentat der Ultrafiltration der wässrigen Lösung oder Suspension verdünnt, dann das verdünnte Retentat mindestens einer Ultrafiltration unterworfen und schließlich ein Retentat gewonnen, das die gewünschte Proteinfraktion enthält. Die Verdünnung des abgetrennten Permeats erfolgt dabei im allgemeinen mit Wasser mit einem Volumen, das kleiner als, so groß wie oder größer als das Volumen des abgetrennten Permeats ist.

Ziel der Erfindung ist ein Verfahren zur Auftrennung von Proteingemischen, wie beispielsweise Blut, Milch, Fermentationsbrühen etc. mittels Membranen. Ein weiteres Ziel der Erfindung ist ein Verfahren, bei dem im kontinuierlichen Prozeß bei totaler Rückhaltung makromolekularer Substanzen Rückgewinnungsraten von > 60 % der niedermolekularen Substanzen erzielt werden.

Überraschenderweise hat sich gezeigt, daß Filtrationsraten von > 70 % bei Verdünnungen von über 10mal, vorzugsweise 20mal erreicht werden können. Hierdurch baut sich keine wesentliche Sekundärmembran auf, so daß auch die in vitro mit Monospezies-Lösungen gemessenen Trenngrenzen der Membran im wesentlichen erhalten bleiben. Die Verdünnungslösung wird dabei zusätzlich zur Rückspülung genutzt, so daß ein nahezu zeitlich konstantes Verhalten der Membran erreicht wird. Die Rückspülung der Substanzen kann durch Druck oder Volumen gesteuert werden.

Das Ziel dieser Erfindung wird somit erreicht mit einem Verfahren zur fraktionierten Trennung von Proteingemischen mittels Membranen bei mindestens zweistufiger Modulanordnung und mehrfacher Verdünnung des zu trennenden Proteingemisches, ausgenommen bei therapeutischer oder chirurgischer Behandlung, welches dadurch gekennzeichnet ist, daß das zu trennende Proteingemisch zunächst mindestens zehnfach verdünnt wird, nach dieser Verdünnung das zu trennende, mindestens zehnfach verdünnte Proteingemisch in eine Filtrationsstufe (1. Modul) gegeben wird, in welcher die hochmolekularen Substanzen bei einer Filtrationsrate von mindestens 70 % abgetrennt werden, und in einer dieser Filtrationsstufe nachgeschalteten weiteren Filtrationsstufe (2. Modul) das Verdünnungsmittel rückgewonnen wird, wobei ebenfalls eine Filtrationsrate von mindestens 70 % eingehalten wird, so daß die niedermolekulare Fraktion in dieser Stufe aufkonzentriert wird und diese Verdünnung, diese Filtrationsstufe und diese weitere Filtrationsstufe kontinuierlich durchgeführt werden.

Besonders vorteilhaft läßt sich die Anordnung bei der fraktionierten Membranplasmapherese einsetzen. Die hohe Filtrationsfraktion der Plasmatrennmembranen bewirkt, daß mehr als 60 % körpereigener Proteine zurückgeführt werden können, so daß kein Fremdprotein benötigt wird.

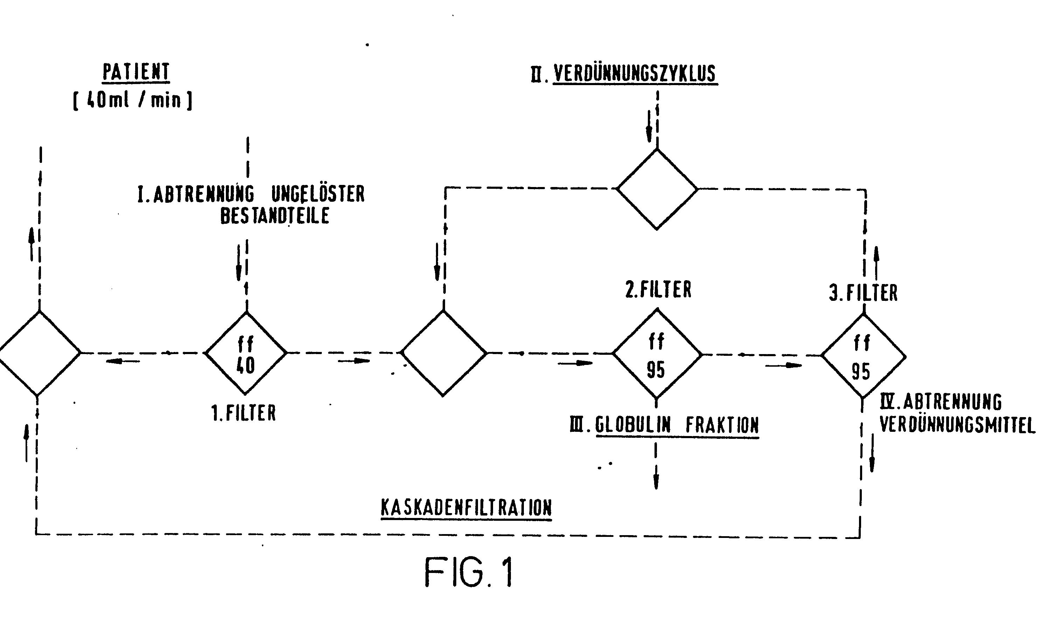

Der integrale Verfahrensablauf wird im folgenden beschrieben. Zur Erläuterung sei auf Figur 1 hingewiesen, in der schematisch der Verfahrensablauf mit Bilanzierung dargestellt ist. Das Verfahren beinhaltet die Stufen :

I. Abtrennung korpuskulärer, kolloidal gelöster oder suspendierter Bestandteile (Vorstufe) ;

II. Verdünnung der in Stufe I gewonnenen Lösung auf > 1 : 10 ;

III. Aufkonzentrieren der makromolekularen Bestandteile bzw. Abtrennung des Verdünnungsmittels zusammen mit den niedermolekularen Bestandteilen bei Filtrationsfraktionen > 70 % ;

IV. Aufkonzentrieren der zurückzuführenden Bestandteile bzw. Abtrennung des Verdünnungsmittels, in welchem Elektrolyte und Moleküle bis zu einer Größe unterhalb der in die Stufe I zurückzuführenden Substanzen enthalten sind. Nicht in Figur 1 dargestellt ist die zur Regeneration der Module in zeitlichen Intervallen durchzuführende Rückspülung, die mit demselben Verdünnungsmittel erfolgt.

Die Kennzeichnung ff in Figur 1 steht für Filtrationsfraktion, die den prozentualen Anteil abgetrennter Lösung von der eingesetzten Lösung darstellt.

Gemäß einem weiteren Aspekt können nach der Stufe IV weitere Stufen eingebaut werden, um eine weitere Fraktionierung der eingesetzten Mischung zu erhalten.

Wesentlich für eine erfolgreiche Durchführung sind die eingesetzten Filter (Module), deren Membranauslegung sich nach der Größe der abzutrennenden Molekülanteile richtet. Hierbei sind die Porengrößen in abnehmender Folge zu wählen.

Das in Figur 1 dargestellte Beispiel stellt die Membrankaskadenplasmapherese dar. Dem Blutkreislauf des Patienten werden während der Behandlung 40 ml/min Blut entnommen. Diese Entnahmerate stellt wiederum nur ein mögliches Beispiel dar ; im praktischen Fall kann sie höher oder niedriger liegen.

Das Blut besteht im Normalfall aus ca. 40 % korpuskulären Bestandteilen und ca. 60 % Blutplasma. Von dem Gesamtvolumen werden 40 % Plasma durch das 1. Filter (Plasmaseparator) nach Verdünnung auf 1 : 20 dem Filter 2 zugeführt. Im 2. Filter wird bei Filtrationsfraktion von 95 % volumenkonstant der hochmolekulare Anteil aufkonzentriert. In diesem Anteil sind je nach durch die Membranmodule festgelegter Trenngrenze alle Bestandteile oberhalb der Trenngrenze enthalten. Im dargestellten Beispiel betrifft dies Moleküle oberhalb Molekulargewicht 150 000. Die Fraktion ist als Globulin-Fraktion bezeichnet. Je nach Krankheitsbild kann sie entweder verworfen werden oder durch erneute Verdünnung und Trennung weiter aufgetrennt werden. Das Ultrafiltrat der III Stufe wird einem nächsten Modul zugeführt, der in diesem speziellen Fall ein Hämofiltrationsmodul ist. In dieser Trennstufe IV wird erneut bei hoher Filtrationsfraktion volumenkonstant der Molekulargewichtanteil oberhalb 60 000 aufkonzentriert, während das Ultrafiltrat dieser Trennstufe in den Verdünnungszyklus zurückgeführt wird. Die Molekülfraktion zwischen MW 60 000 und MW 150 000, in der das Humanalbumin und ein großer Teil der Gerinnungsfaktoren enthalten sind, wird zusammen mit dem Konzentrat der Stufe I dem Patienten zurückgegeben.

Die beispielhaft durchgeführte Bilanzierung zeigt, daß das Verfahren volumenkonstant arbeitet, wobei dem Patienten keine körperfremden Substanzen außer einem kleinen Anteil Elektrolytlösung zurückgegeben werden. Bei den hier gewählten und durch das Verfahren einstellbaren Bedingungen ist eine Behandlung von 2 bis 3 Stunden ausreichend, um 6-7 l Blut zu filtrieren.

Im zeitlichen Verlauf der Fraktionierung wird die Volumenkonstanz dadurch erreicht, daß mit fest eingestellten Zuführungs- bzw. Filtrationsmengen gearbeitet wird, wobei der Druck im Modul durch den sich erhöhenden Membranwiderstand ansteigt. Ist ein festgelegter Maximaldruck erreicht — im Beispiel 500 Torr (666 mbar) — wird die Rückspülung mit dem Verdünnungsmittel durchgeführt. Hierbei geht der Betriebsdruck wieder auf den Anfangswert zurück.

# 0 113 447

Die im folgenden aufgeführten Beispiele sollen das Verfahren näher erläutern, ohne daß hiermit eine Einschränkung der Anwendungsbreite gegeben wird :

1. Beispiel

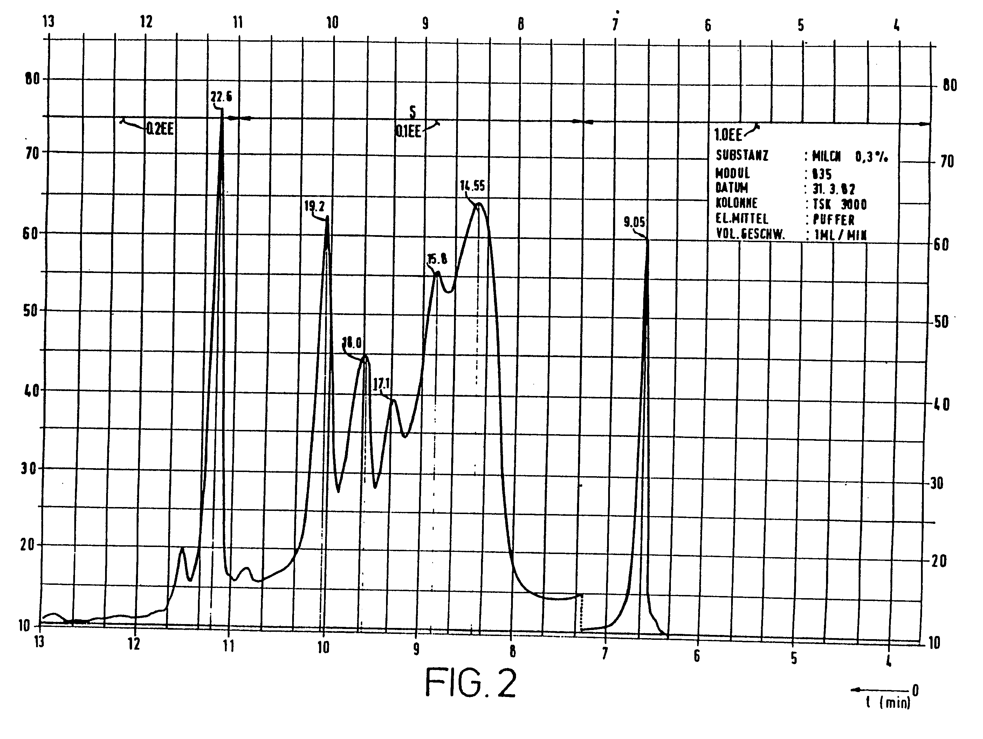

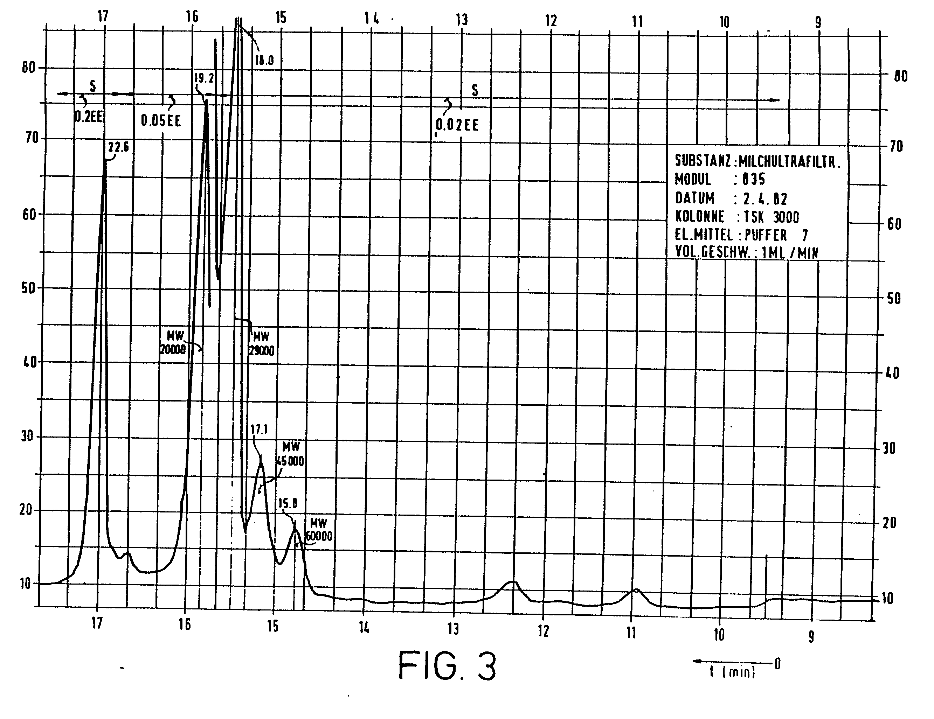

Milch der Stufe Magermilch mit 0,3 % Fettgehalt wurde in unterschiedliche Bestandteile zerlegt. Hierbei wurde ein Modul eingesetzt, dessen Kapillarmembranen bei einem inneren Durchmesser von 282 μm eine Wandstärke von 57 μm aufweisen. Das Porenvolumen der Membran betrug 0.6 ml/g, der mittlere Porenradius 12.4 nm. Die eingesetzte Milch wies eine Zusammensetzung auf, die durch das HPLC-Chromatogramm (Fig. 2) charakterisiert ist. Nach Abtrennung der kolloidalen und höhermolekularen Bestandteile (> 68 000 Dalton) wurde die Charakterisierung wie in Figur 3 dargestellt erhalten. Kennziffern hieraus sind :

Durchgang MW 68 000 : 6.8 %
45 000 : 18.8 %
29 000 : 53.5 %
20 000 : 71.4 %

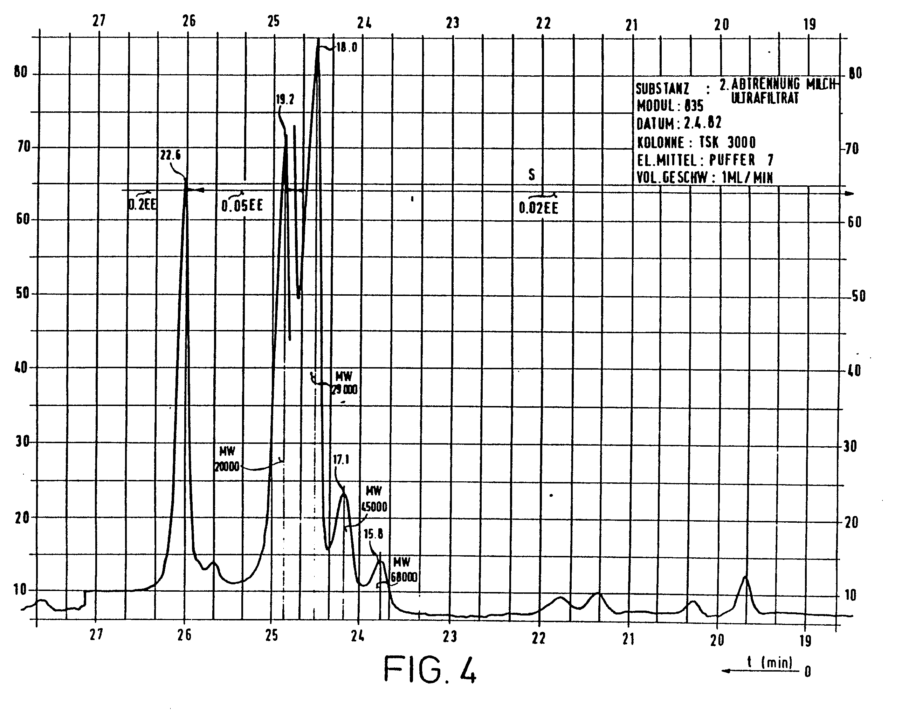

Das in der Aufkonzentrierungsstufe eingesetzte Permeat zeigte die Werte (s. Fig. 4) :

Durchgang MW 68 000 : 79.2 %
45 000 : 85.4 %
24 000 : 93.5 %
20 000 : 94.7 %

Bemerkenswert an diesem Beispiel ist, daß mit einer Modulart sämtliche Abtrennungsschritte durchgeführt wurden. Der Unterschied in der Trennwirkung wurde allein durch die unterschiedliche Konzentration der vorliegenden Substanzen erzielt.

2. Beispiel

Blut des Krankheitsbildes Dermatomysites wurde fraktioniert. Nach Abtrennung der korpuskulären Bestandteile und bei einer — in Stufe II des in Figur 1 dargestellten Verfahrensschemas-Verdünnung von 1 : 20 wurde das verdünnte Plasma der Stufe III zur weiteren Auftrennung zugeführt und danach in Stufe IV aufkonzentriert. Bezogen auf die Einzelsubstanzen wurde dabei folgende Ausbeute erreicht :

| Substanz | eingesetzt [ mg/min ] | gewonnen [ mg/min ] | Ausbeute [ % ] |
|---|---|---|---|
| Albumin | 66.94 | 45.12 | 67.4 |
| Immunglobulin G | 8.45 | 5.21 | 62 |
| Immunglobulin A | 1.17 | 0.46 | 39 |
| Immunglobulin M | 1.77 | nicht meßbar | 0 |

3. Beispiel

Blut des Krankheitsbildes IGA Plasmozytom wurde entsprechend Beispiel 2 behandelt. Die Verdünnung betrug hierbei 1 : 14.

| Substanz | eingesetzt [ mg/min ] | gewonnen [ mg/min ] | Ausbeute [ % ] |
|---|---|---|---|
| α₁ Antitrypsin | 1.78 | 1.08 | 61 |
| Albumin | 21.12 | 12.67 | 60 |
| Transferrin | 1.48 | 0.38 | 26 |
| Immunglobulin G | 1.42 | 0.75 | 53 |

4

**0 113 447**

(Fortsetzung)

| Substanz | eingesetzt [mg/min] | gewonnen [mg/min] | Ausbeute [%] |
|---|---|---|---|
| Immunglobulin A | 32.99 | 12.47 | 38 |
| Caeruloplasmin | 0.15 | nicht meßbar | 0 |
| Komplement C₄ | 0.17 | 0.05 | 29 |
| Komplement C₃ | 0.43 | 0.14 | 33 |
| Immunglobulin M | 0.13 | nicht meßbar | 0 |

Die deutliche Reduktion des Immunglobulin A um 62 % bei einer Albuminrückgewinnung von 60 %, gemittelt über die gesamte Behandlungszeit, demonstriert die Vorteile dieses Verfahren gegenüber dem derzeitigen Stand der Technik.

Das erfindungsgemäße Verfahren ist geeignet zur Auftrennung von Proteingemischen aller Art. Es ist beispielsweise sehr gut geeignet zur Aufarbeitung von Fermentationsbrühen, wobei das Endprodukt von dem Enzym, von anderen hochmolekularen Stoffen und von korpuskularen Bestandteilen abgetrennt werden soll. Beispielsweise kann das erfindungsgemäße Verfahren mit großem Vorteil einem Enzymreaktor nachgeschaltet werden.

**Patentansprüche**

1. Verfahren zur fraktionierten Trennung von Proteingemischen mittels Membranen bei mindestens zweistufiger Modulanordnung und mehrfacher Verdünnung des zu trennenden Proteingemisches, ausgenommen die Trennung bei therapeutischer oder chirurgischer Behandlung dadurch gekennzeichnet, daß

das zu trennende Proteingemisch zunächst mindestens zehnfach verdünnt wird,

nach dieser Verdünnung das zu trennende, mindestens zehnfach verdünnte Proteingemisch in eine Filtrationsstufe (1. Modul) gegeben wird, in welcher die hochmolekularen Substanzen bei einer Filtrationsrate von mindestens 70 % abgetrennt werden,

in einer dieser Filtrationsstufe nachgeschalteten weiteren Filtrationsstufe (2. Modul) das Verdünnungsmittel rückgewonnen wird, wobei ebenfalls eine Filtrationsrate von mindestens 70 % eingehalten wird, so daß die niedermolekulare Fraktion in dieser Stufe aufkonzentriert wird, und

diese Verdünnung, diese Filtrationsstufe und diese weitere Filtrationsstufe kontinuierlich durchgeführt werden.

2. Verfahren nach Anspruch 1, dadurch gekennzeichnet, daß mit mindestens zwanzigfacher Verdünnung gearbeitet wird.

3. Verfahren nach Anspruch 1, dadurch gekennzeichnet, daß eine Vorstufe vorgeschaltet wird, in welcher ohne Verdünnung eine Abtrennung ungelöster Bestandteile mit Filtrationsraten von maximal 60 % durchgeführt wird.

4. Verfahren nach einem der Ansprüche 1 bis 3, dadurch gekennzeichnet ; daß die Verdünnungslösung in zeitlich oder mengenmäßig festgelegten Abständen durch die Membran rückgespült wird.

5. Verfahren nach einem der Ansprüche 1 bis 3, dadurch gekennzeichnet, daß eine mittels der Verdünnungslösung durchgeführte Rückspülung der Membran druckgesteuert erfolgt.

6. Verfahren nach einem der Ansprüche 1 bis 3, dadurch gekennzeichnet, daß eine mittels der Verdünnungslösung durchgeführte Rückspülung der Membran volumengesteuert erfolgt.

7. Anwendung des Verfahrens gemäß den Ansprüchen 1-6 zur fraktionierten Membranplasmapherese.

8. Anwendung des Verfahrens gemäß den Ansprüchen 1 bis 6 zur Aufarbeitung von Fermentationsbrühen.

**Claims**

1. A process for the fractional separation of protein mixtures by means of membranes, with modules being arranged at least in two stages and multiple dilution of the protein mixture to be separated, except the separation in therapeutical or surgical treatment, characterized by

diluting the protein mixture to be separated firstly at least ten fold,

introducing, after this dilution, the protein mixture to be separated and diluted at least ten fold into a

5

filtration stage (1st module), wherein the high molecular weight substances are separated at a filtration rate of at least 70 percent,

recovering the dilution medium in a further filtration stage (2nd module), being connected behind this filtration stage, also maintaining a filtration rate of at least 70 percent to concentrate the low molecular fraction in this stage, and

performing this dilution, this filtration stage and this further filtration stage continuously.

2. A process according to claim 1, characterized by operating at least a twenty-fold dilution.

3. A process according to claim 1, characterized in that a preceding stage is connected ahead in which a separation of undissolved ingredients is made at filtration rates of at most 60 percent without dilution.

4. A process according to one of the claims 1 to 3, characterized in that the dilution medium is backwashed through the membrane at predetermined intervals with respect to time or to the amount of the dilution medium.

5. A process according to one of the claims 1 to 3, characterized in that a pressure-controlled backwash of the membrane is performed by means of the dilution medium.

6. A process according to one of the claims 1 to 3, characterized in that a volume-controlled backwash of the membrane is performed by way of the dilution medium.

7. The use of the process according to claims 1 to 6, for fractional membrane plasmaphoresis.

8. The use of the process according to claims 1 to 6 for the reconditioning of fermentation liquors.

## Revendications

1. Procédé pour la séparation fractionnée de mélanges de protéines à l'aide de membranes dans un dispositif comportant au moins deux étages de modules et une dilution de plusieurs fois du mélange des protéines à séparer, à l'exclusion de la séparation pour un traitement thérapeutique ou chirurgical, procédé caractérisé en ce que :

on dilue tout d'abord d'au moins dix fois le mélange des protéines à séparer,

après cette dilution, on fait passer le mélange des protéines à séparer, dilué d'au moins dix fois, par une étape de filtration (premier module), dans laquelle les substances à poids moléculaire élevé sont séparées à un taux de filtration d'au moins 70 %,

dans une autre étape de filtration (second module), placée après cette étape de filtration, on récupère l'agent de dilution, en maintenant également un taux de filtration d'au moins 70 %, de sorte que la fraction à bas poids moléculaire est concentrée dans cette étape, et

on effectue en continu cette dilution, cette étape de filtration et cette nouvelle étape de filtration.

2. Procédé selon la revendication 1, caractérisé en ce qu'on travaille avec une dilution d'au moins vingt fois.

3. Procédé selon la revendication 1, caractérisé en ce qu'il comporte une étape préliminaire dans laquelle on effectue, sans dilution, à des taux de filtration d'au maximum 60 %, une séparation des constituants non dissous.

4. Procédé selon l'une quelconque des revendications 1 à 3, caractérisé en ce qu'on utilise, pour un rinçage à contre-courant, la solution de dilution à des intervalles déterminés dans le temps ou selon les quantités utilisées.

5. Procédé selon l'une quelconque des revendications 1 à 3, caractérisé en ce qu'un rinçage à contre-courant de la membrane, effectué à l'aide de la solution de dilution, est effectué sous la commande de la pression appliquée.

6. Procédé selon l'une quelconque des revendications 1 à 3, caractérisé en ce qu'un rinçage à contre-courant de la membrane, effectué à l'aide de la solution de dilution, est effectué avec commande par les volumes.

7. Application du procédé selon l'une quelconque des revendications 1 à 6 pour une plasmaphérèse fractionnée sur des membranes.

8. Application du procédé selon l'une quelconque des revendications 1 à 6 pour un traitement de purification de bouillons de fermentation.

FIG.1

FIG.2

FIG. 3

SUBSTANZ: MILCHULTRAFILTR.
MODUL    : 835
DATUM    : 2.4.82
KOLONNE  : TSK 3000
EL.MITTEL: PUFFER 7
VOL.GESCHW.: 1ML/MIN

t (min)

0 1 1 3 4 4 7

FIG. 4